(19) Europäisches Patentamt
European Patent Office
Office européen des brevets

(11) **EP 0 713 454 B1**

(12) **EUROPÄISCHE PATENTSCHRIFT**

(45) Veröffentlichungstag und Bekanntmachung des Hinweises auf die Patenterteilung:
**12.03.1997 Patentblatt 1997/11**

(21) Anmeldenummer: **94926206.7**

(22) Anmeldetag: **18.08.1994**

(51) Int. Cl.$^6$: **B60H 3/00**

(86) Internationale Anmeldenummer:
**PCT/EP94/02742**

(87) Internationale Veröffentlichungsnummer:
**WO 95/05949 (02.03.1995 Gazette 1995/10)**

(54) **SIGNALAUSWERTUNG VON SCHADSTOFFSENSOREN**

POLLUTION SENSOR SIGNAL EVALUATION

EVALUATION DE SIGNAUX DE CAPTEURS POLLUANTS

(84) Benannte Vertragsstaaten:
**DE ES FR GB IT SE**

(30) Priorität: **21.08.1993 DE 4328218**

(43) Veröffentlichungstag der Anmeldung:
**29.05.1996 Patentblatt 1996/22**

(73) Patentinhaber: **I.T.V.I. INTERNATIONAL TECHNO VENTURE INVEST AG**
**9490 Vaduz (LI)**

(72) Erfinder:
• **RUMP, Hanns**
**D-59427 Unna (DE)**

• **HILLER, Jörg**
**D-58300 Wetter (DE)**
• **PIEPER, Norbert**
**D-59379 Selm (DE)**

(74) Vertreter: **Spalthoff, Adolf, Dipl.-Ing. et al**
**Spalthoff, Adolf, Dipl.-Ing.**
**Lelgemann, Karl-Heinz, Dipl.-Ing.,**
**Postfach 34 02 20**
**45074 Essen (DE)**

(56) Entgegenhaltungen:
**EP-A- 0 065 371**          **EP-A- 0 501 127**
**WO-A-88/02704**          **DE-A- 3 731 745**
**US-A- 5 062 065**

**Beschreibung**

Nachfolgend beschriebene Erfindung beschreibt den Einsatz von Sensoren zum Zwecke der Auslösung eines elektrischen Impulses dann, wenn erhöhte Konzentrationen von Gasen auftreten, für die das oder die Sensorelement(e) ausgelegt sind (= Zielgase).
In einer Anwendung soll z. B. die Luftzufuhr zur Kabine eines Fahrzeuges dann unterbrochen werden, wenn durch Emmissionen anderer Fahrzeuge etc. eine erhöhte Konzentration von Gasen wie Kohlenmonoxid, Stickoxiden, Schwefeldioxid, teilverbrannten Kohlenwasserstoffen etc. auftreten.
Bekannt ist, zu diesem Zweck verschiedene Sensoren einzusetzen, z. B.:

| | |
|---|---|
| Zinndioxid ($SnO_2$) | für alle oxidierbaren Gase |
| Phthalocyanin | für reduzierbare Gase |
| Wolframtrioxid ($WO_3$) | für Stickoxide |

Zur Auswertung der Signale derartiger Sensoren ist bekannt, nicht bei Erreichen eines absoluten Pegels das Schaltsignal auszulösen, sondern dann, wenn relativ zum Durchschnittspegel eine erhöhte Konzentration der Zielgase vom Sensor, bzw. den Sensoren detektiert wird.

Da Sensoren einer gewissen Drift unterliegen, meist querempfindlich gegenüber der Luftfeuchte sind und in Regionen unterschiedlichster Grundbelastung mit Schadstoffen zu betreiben sind, ist vorgeschlagen worden, das Sensorsignal über einen elektrischen Hochpaß zu differenzieren, wobei die von anderen Fahrzeugen herrührenden Schadstoffwerte meist zu deutlich schnelleren Schaltimpulsen führen als o. g. Einflüsse. Wird die Grenzfrequenz des Hochpasses so gewählt, daß diese Impulse (Zielimpulse) im Gegensatz zu den langsameren Veränderungen übertragen werden, wird vorteilhaft jede relevante Verschlechterung des Schadstoffpegels zu einem Schaltsignal führen. Es ist weiter vorgeschlagen worden, das Signal mehrfach zu diffenenzieren, um auch kleinere Signaländerungen auswerten zu können (siehe EP-A-87 00 592).

Da Sensoren in der Regel schnell auf ein Zielgas reagieren, aber signifikant langsamer wieder den Ursprungswert annehmen, kann mit dieser Methode nur vorteilhaft der Befehl "Luftzufuhr unterbrechen", jedoch weniger gut der Aufhebungsbefehl "Luftzufuhr wieder öffnen" erzeugt werden.
Bekannt ist es, den Mittelwert des Sensorsignals über eine gewisse Zeit (z. B. 10 Minuten) fortlaufend zu bilden und den aktuellen Sensorwert mit diesem "gelernten" Mittelwert zu vergleichen. Daraus läßt sich sowohl das Signal "Luftzufuhr sperren" als auch das Signal "Luftzufuhr wieder öffnen" gut ableiten. Details sind in der EP-A-87 00 592 beschrieben. Allerdings sind für dieses Verfahren Sensorsignale einer gewissen Amplitude erforderlich. Bei sehr kleinen Sensorsignalen kommt es zu Unsicherheiten in der Auswertung der Signale.

Erfindungsgemäß wird daher das im Patentanspruch 1 angegebene Verfahren vorgeschlagen, bei dem die Änderungen der Signale als solche zur Auswertung herangezogen werden, wobei der Betrachtung des zeitlichen Verhaltens eine wesentliche Rolle spielt.

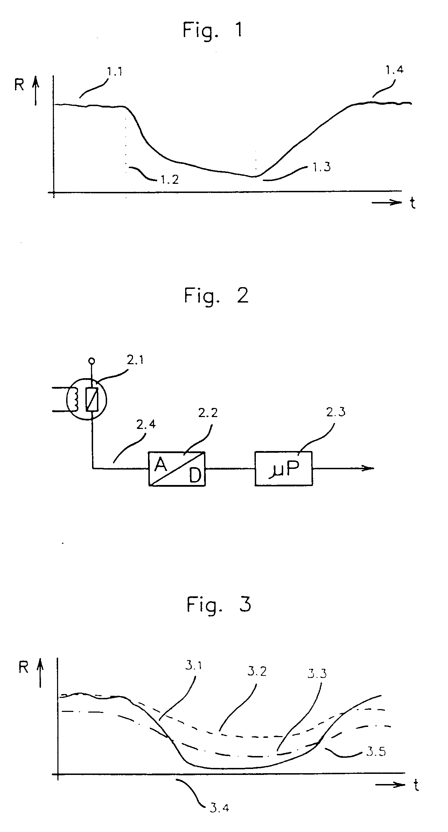

Fig. 1 zeigt das typische Sensorsignal im Falle der Provokation mit Zielgasen. Bei 1.1 ist der Sensor wenig belastet (Normalluft-Wert). Die Belastung der Luft mit Zielgasen beginnt zum Zeitpunkt 1.2. Hier ist ein negativer Gradient sicher zu detektieren und in seiner Steigung deutlich größer als normale Schwankungen. Während der Anwesenheit von Zielgasen ist der Sensor im Bereich 1.2. Normalisiert sich die Luftbelastung wieder, ist ein Anstieg des Sensorwertes zuerst bei 1.3 zu beobachten, wobei nach einiger Zeit der Ausgangswert 1.4 erreicht wird.

Es wäre sinnvoll, zum Zeitpunkt 1.2 die Luftzufuhr zu unterbrechen und zum Zeitpunkt 1.3 diese wieder zu ermöglichen.
(Im Beispiel ist ein Sensor beschrieben, der bei Gaseinwirkung niedere elektrische Widerstände annimmt.
Prinzipiell ist das Ausgeführte auch gültig, wenn der Sensor eine umgekehrte Charakteristik hat, also bei Gaseinwirkung hochohmiger wird.)

In der Praxis sieht das Sensorsignal allerdings keineswegs so eindeutig aus, so daß an die Signalverarbeitung höhere Anforderungen zu stellen sind.

In der bevorzugten Ausführung (Fig. 2) erfolgt die Auswertung des Sensorsignals 2.4 in einem programmgesteuerten Microcomputer 2.3, wobei die Signale des Sensors 2.1 in einem Analog-/Digitalwandler 2.2 digitalisiert werden.

Fig. 3 zeigt stark vereinfacht das erfindungsgemäße Prinzip: Dabei ist 3.1 das Sensorsignal, welches naturbedingt kleinere Schwankungen zeigt. 3.2 ist ein rechnerischer Durchschnittswert (Integral) des Signals 3.1, wobei die Integrationszeit frei bestimmt werden kann und in der Regel 5 - 10 Min. ist.
3.3 ist eine vom Rechner gebildete Kurve, die jeweils um einen geringen Betrag (=x) geringere numerische Werte als die Kurve 3.2 hat. x kann ein Festbetrag sein oder aber ein Proportionalbetrag von z. B. 1 %.

Die Luftzufuhr wird zum Zeitpunkt 3.4 unterbrochen, wenn die Kurve 3.1 einen kleineren Wert annimmt als die Kurve 3.3 und wird zum Zeitpunkt 3.5 wieder geöffnet, wenn der Wert 3.1 wieder größer wird als der Wert 3.3. Es ist dabei erkennbar, daß die Integrationszeit einen erheblichen Einfluß hat.

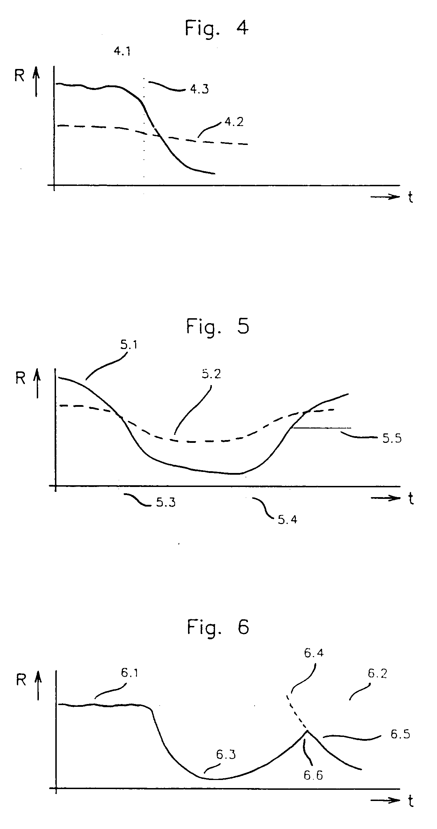

Ist die Integrationszeit relativ klein, würde die Lüftung bereits bei objektiv noch hohen Schadstoff-Belastungen öffnen. Ist die Integrationszeit dagegen sehr groß, wird auch die Schließzeit in manchen Situationen subjektiv zu groß sein.
Sehr große Konzentrationen der Zielgase erzeugen im Moment der Detektion bei den Sensoren eine sehr schnelle Signaländerung. Vorteilhaft schlägt die Erfindung für diese Situation vor, zusätzlich stets dann ein Schaltsignal auszulösen, wenn der Differential-Betrag des Sensorsignals über die Zeit größer ist als ein vorbestimmter Wert (y), unabhängig davon, ob bereits die vorstehend in Fig. 3 beschriebene Schaltbedindung erfüllt ist. Fig. 4 beschreibt das Prinzip:

Der Sensorwert 4.1 erfährt unter Begasung mit dem Zielgas eine schnelle Änderung. Bevor er die Schwelle 4.2 erreicht, wird ein

$$\frac{\Delta s}{\Delta t} > y$$

vom Auswerterechner 2.3 festgestellt. Damit ist eine weitere Schaltbedingung erfüllt und der Schaltimpuls wird ausgelöst.

Bei Erläuterung der Fig. 3 war angemerkt, daß die Aufhebung des Schließbefehls, also der Befehl "Luftzufuhr zulassen" mit der beschriebenen Methode zwar funktioniert, physiologisch und sachlich aber in manchen Situationen unbefriedigend ist.

Fig. 5 zeigt eine vorteilhafte Lösung. Dabei ist 5.1 der Verlauf des aktuellen Sensorsignals und 5.2 ist das Integral des Sensorsignals minus des Betrages x. Zum Zeitpunkt 5.3 wird der Luftzutritt unterbrochen. Zum Zeitpunkt 5.4 ist erkennbar, daß sich der Sensorwert mit positivem Gradienten verbessert. Das Kriterium für die Öffnung der Luftzufuhr ist erfüllt, wenn der aktuelle Sensorwert ausgehend vom Sensorwert im Zeitpunkt 5.4 einen um den Betrag y erhöhten Wert 5.5 erreicht. Vorteilhaft wird durch diesen erfindungsgemäßen Vorschlag erreicht, daß die Integrationszeit für das Schaltverhalten nicht mehr die alleinige Gestaltungsgröße ist. Die Erfindung macht sich vorteilhaft die Beobachtung zu nutze, daß sich das menschliche Riechorgan an "schlechte Luft" anpassen kann und wesentlich nur auf Veränderungen der Luftzusammensetzung bzw. der Luftbelastung reagiert.

Reagiert die erfindungsgemäße Anordnung sozusagen auf einen "Frischluftimpuls", unabhängig von der objektiven Qualität der Luft, wird dies von der menschlichen Nase als plausibel empfunden werden. Um diese Plausibilität weiter zu verbessern, ist das in Fig. 6 beschriebenen erfindungsgemäße Prinzip vorgeschlagen worden. In unbelasteter Luft hat der Sensor den Wert 6.1 angenommen. Nach Kontakt mit dem Zielgas wird der Wert 6.3 erreicht. Gestrichelt ist der Ausgangswert (Frischluft-Pegel) als 6.2 weitergezogen. Nunmehr wird ein weiterer Schadstoffimpuls auf den Sensor gegeben und erzeugt die Reaktion 6.5. Die auf den ersten Blick unscheinbare Reaktion stellt in Wirklichkeit das Ergebnis eines Schadstoffimpulses, ähnlich 6.3 dar. Allerdings traf dieser Impuls 6.5 auf einen in der Desorptionsphase 6.6 befindlichen Sensor. Wäre der Ausgangspunkt der Frischluftpegel 6.2 gewesen, wäre ein Signal ähnlich 6.4 zu registrieren gewesen, dessen großer Gradient das in Fig. 4 beschriebene Schaltkriterium erfüllt hätte.

Erfindungsgemäß wird daher vorgeschlagen, daß der Rechner 2.3 jederzeit den Wert und die Richtung der jeweiligen Steigung feststellt. In der Desorptionsphase 6.6 wird ein positiver Änderungs-betrag von D1 festgestellt. Im Moment des Gasimpulses kommt es zu einer Richtungsumkehr und der nun negative Änderungsbetrag ist bei 6.5 gleich D2. Das Rechenprogramm addiert nunmehr die Absolutwerte der beiden Änderungsbeträge

$$abs (D1) + abs (D2) = D3$$

D3 ist der zu bewertende Änderungsbetrag. Das Programm prüft nunmehr, ob der Änderungsbetrag größer als y ist, was unverzüglich zum Sperren der Luftzufuhr führen würde. Je nach der Charakteristik des verwendeten Sensorelementes und insbesondere seiner Trägheit in der Desorptionsphase 6.6 wird vorgeschlagen, den Änderungsbetrag D1 mit einem Faktor zu multiplizieren, um mit D3 einen Wert zu erhalten, der dem Änderungsbetrag 6.4 im Falle des nicht desorbierenden Sensors entspricht.

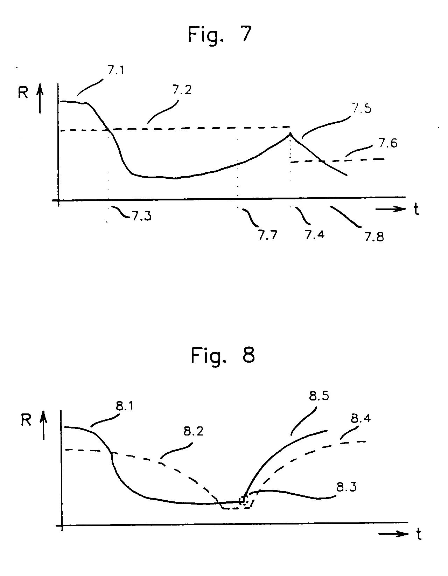

Mit der vorstehend beschriebenen Lehre wird in den meisten Fällen ein befriedigendes Schaltverhalten erreicht. Insbesondere dann, wenn in kurzer Folge Impulse der Zielgase auf den (die) Sensor(en) gegeben werden, kommt es nicht immer zu befriedigendem und plausiblem Schaltverhalten. Aus diesem Grunde wird entsprechend Fig. 7 wie folgt vorgeschlagen:

Der Sensorausgangswert ist 7.1, der sich unter Gaseinfluß vermindert und zum Zeitpunkt 7.3 die aus dem Integral gebildeten Wert 7.2 unterschreitet und damit das Schaltkriterium erfüllt. Ohne Zielgas erholt sich der Sensor wieder und zeigt einen positiven Gradienten, der zum Zeitpunkt 7.7 den Wert y erreicht hat, was den Schaltimpuls (Luftzufuhr öffnen) auslöst. Zum Zeitpunkt 7.4 wird eine Umkehr des Gradienten festgestellt, was auf ein erneutes Auftreten des Zielgases schließen läßt.

In diesem Moment wird der Vergleichswert von 7.2 auf den Wert 7.6 gesetzt, welches der aktuelle Sensorwert zum Zeitpunkt 7.4, vermindert um den aus Fig. 3 bekannten Betrag x, ist. Zum Zeitpunkt 7.8 durcheilt der aktuelle Sensorbetrag die Kurve 7.6 und löst dabei das Schaltsignal "Luftzufuhr unterbrechen" erneut aus.

In einer weiteren Ausformung des erfindungsgemäßen Gedankens wird entsprechend Fig. 8 die Bildung des Vergleichwertes 8.2 und 8.4 vom Betrag und der Richtung des Gradienten abhängig gemacht, den der aktuelle Sensorwert 8.1 und 8.5 annimmt. Grund ist die Beobachtung, daß manche Sensorprinzipien ein gegen der Adsorptions- deutlich längere Desorptionsphase haben.

In der Desorptionsphase findet die Integration mehrheitlich sozusagen im Sensor selbst statt, so daß eine weitere Integration das Nachführen des Vergleichwertes zu langsam macht. Erfindungsgemäß wird daher vorgeschlagen, den Vergleichswert 8.4 nach der Richtungsumkehr des Gradienten 8.3 entweder direkt aus dem aktuellen Sensorwert, vermindert um einen Betrag x oder aber aus einem Integral mit verkleinerter Integrationszeit zu bilden. Wird der Betrag des Gradienten kleiner als ein frei bestimmter Wert, erfolgt wieder die Integration der Sensorwerte mit längerer Zeitspanne.

Die Lehre der Erfindung wird bevorzugt in einer Version nach Fig. 2 angewandt werden, wobei die Ausführung einem pragrammgesteuerten Mikroprozessor als rein rechnerische Operation zugewiesen ist.

Selbstverständlich ist es dem Fachmann mit einigem Aufwand auch möglich, das beschriebene Auswerteverfahren auch rein analogtechnisch mit üblichen elektronischen Bausteinen zu realisieren. Die Formulierung der nachfolgenden Patentansprüche beschreiben aus diesem Grunde jeweils die Schaltbedingungen und die logischen Verknüpfungen, unabhängig von der zur Ausführung eingesetzten Technik. Es wird ausdrücklich darauf hingewiesen, daß die vorgeschlagene Technik unabhängig von der Anzahl der eingesetzten Sensorelemente oder der Art der eingesetzten Sensortechnologie ist.

**Patentansprüche**

1. Verfahren zur Auswertung von Sensorsignalen mit dem Ziel, Schaltimpulse zu erzeugen, die die Luftzufuhr eines Innenraumes, vorzugsweise des Innenraums eines Kraftfahrzeuges über geeignete Stellglieder und Luftführungen so beeinflussen, daß die Luftzufuhr bei Auftreten einer ein Schaltkriterium darstellende Gaskonzentration unterbrochen wird oder, daß die zutretende Luft vor Erreichen des Innenraumes über ein Filtersystem geführt wird,
und, daß der ursprüngliche Zustand dann wieder hergestellt wird, wenn die Qualität der Außenluft sich wieder verbessert hat, dadurch gekennzeichnet,
daß entsprechend Fig. 3 bei Sensoren, die bei Gasangebot ihren elektrischen Widerstand vermindern, aus dem aktuellen Sensorwert 3.1 ein über eine frei bestimmte Zeit (t) ein Integral 3.2 gebildet wird, das um einen Betrag (x) vermindert wird und insofern den Wert 3.3 annimmt,
wobei die freie Luftzufuhr dann unterbrochen wird, sobald 3.4 der aktuelle Sensorbetrag 3.1 den errechneten Betrag 3.3 unterschreitet, oder daß dann, wenn die Auswertelektronik 2.3 feststellt, daß entsprechend Fig. 4 der Änderungsbetrag des Sensorsignals 4.3 in einer frei bestimmbare Zeit größer als ein frei bestimmter Wert (y) ist, ebenfalls ein Schaltsignal ausgelöst wird.

2. Verfahren nach Anspruch 1, dadurch gekennzeichnet,
daß im Falle von Sensoren, die bei Gasangebot ihren elektrischen Widerstand vergrößern, die in Anspruch 1 genannten Regeln sinngemäß gelten, wobei entsprechend Figur 8 das Integral 8.2 über dem Sensorwert 8.1 liegt und der Änderungsbetrag (y) einem positiven Gradienten zugeordnet ist.

3. Verfahren nach Anspruch 1, dadurch gekennzeichnet,
daß die freie Luftzufuhr dann durch Rücknahme des in Anspruch 1 und 2 erzeugten Schaltbefehls wieder ermöglicht wird, wenn entsprechend Figur 3 das aktuelle Sensorsignal 3.1 wieder einen größeren Betrag annimmt als das um den Betrag (x) verminderte Integral 3.3.

4. Verfahren nach Anspruch 1, dadurch gekennzeichnet,
daß die freie Luftzufuhr dann durch Rücknahme des in Anspruch 1 oder 2 erzeugte Schaltbefehls wieder ermöglicht wird, wenn entsprechend Fig. 5 die Tendenz des Sensorsignals 5.1 sich um Zeitpunkt 5.4 durch Desorption umkehrt und in Auswerteelektronik das zu diesem Zeitpunkt aktuelle Sensorsignal gespeichert wird und um einen festen oder proportionalen Betrag zu einem Schaltpegel 5.5 erhöht wird, wobei das erwähnte Schaltsignal dann ausgelöst wird, wenn der Wert des aktuellen Sensorsignals den Betrag 5.5 überschreitet. Das gilt für Sensoren, die auf Gasangebot mit einer Verringerung des Sensorwiderstandes reagieren.

5. Verfahren nach Anspruch 4, dadurch gekennzeichnet,
daß bei einem Sensor, der auf Gasangebot mit Erhöhung des Sensorwiderstandes reagiert, daß im Anspruch 4 gesagte sinngemäß gilt, wobei die Pegelverhältnisse invers sind.

6. Verfahren nach Anspruch 1, dadurch gekennzeichnet,
daß entsprechend Fig. 7 nach Aufheben des Schließbefehls zum Zeitpunkt 7.7 der Gradient überwacht wird und jeweils der zuletzt gültige Quotient aus Änderungsbetrag des Sensorpegels und einem bestimmten Zeitabschnitt gespeichert wird.
Bei Umkehr des Gradienten 7.4 wird der negative Gradient D1 7.5 als Absolutwert mit dem Absolutwert des positiven Gradienten D2 7.7 addiert, wobei es dann zum Auslösen eines Schaltimpulses kommt, wenn der Summenwert beider Gradienten größer als ein bestimmter Wert D3 ist. Zum Zeitpunkt 7.4 wird der durch einen Betrag (x) verminderte integrierte Sensorwert aus den Sensorwerten einer frei definierbaren zurück-liegenden Zeitspanne 7.2 von der Auswerteeinheit 2.3 auf einen neuen Wert gesetzt, der sich aus dem Sensorwert zum Zeitpunkt der Gradientenumkehr 7.4, vermindert durch den Betrag (x) ergibt, wobei die ab dem Zeitpunkt 7.4 ermittelten neuen Sensorwerte zur Bildung eines neuen Integrals 7.6 herangezogen werden. Als Schaltkriterium gilt nach der Gradientenumkehr entweder

D3 ist größer als y

oder

der aktuelle Sensorwert wird kleiner als die wie vorstehend neu definierte Kurve 7.6.

Zellewerte einer frei definierbaren zurückliegenden

Im Falle der Verwendung von Sensoren, die ihren Widerstand bei Gasangeboten erhöhen, gilt das ausgeführte sinngemäß, wobei die Pegelveränderungen allerdings invers sind.

7. Verfahren nach den Ansprüchen 1 - 6, dadurch gekennzeichent,
daß entsprechend Fig. 8 die Bildung eines Vergleichspegels aus dem integrierten Wert der Einzelwerte einer frei definierbaren zurückliegenden Zeitspanne, vermindert um einen Betrag x 8.2 ausgesetzt wird, wenn der Gradient des aktuellen Sensorwertes 8.4 positiv wird. Ist der Gradient positiv, bildet sich der Vergleichswert 8.4 aus dem aktuellen Sensorwert, abzüglich eines Betrages x.
Im Falle der Verwendung von Sensoren, die ihren Widerstand bei Gasangebot erhöhen, gilt das gesagte sinngemäß, wobei die Pegelveränderungen jedoch invers sind.

8. Verfahren nach Anspruch 7, dadurch gekennzeichnet,
daß der Vergleichspegel 8.2 sich aus dem Integral von Sensorwerten einer bestimmten Zeitspanne ergibt, abzüglich eines Betrages x und das nach der Gradientumkehr zum Zeitpunkt 8.3 die Integrationsdauer verkürzt ist. Die normale, längere Integrationszeit wird wieder erreicht, wenn der Gradient 8.5 einen Quotienten erreicht, der kleiner als ein bestimmter Wert ist.

9. Verfahren nach mindestens einem der vorangegangenen Ansprüche, dadurch gekennzeichnet,
daß die Merkmale eines der Ansprüche mit einem anderen Merkmal eines der anderen Ansprüche kombiniert ist.

**Claims**

1. Process for the evaluation of sensor signals with the object of generating switching pulses, which influence the air supply of an inner chamber, preferably the inner chamber of a vehicle, via suitable setting members and air ducts in such a manner that the air supply is interrupted if a gas concentration which constitutes a switching condition occurs, or that the incoming air is conducted via a filter system prior to reaching the inner chamber; and in that the original state is produced again when the quality of the external air has improved again, characterised in that, as shown in Figure 3, in the case of sensors which reduce their electronic resistance when exposed to gas, an integral 3.2 is formed of the momentary sensor value 3.1 in a freely determined time (t), which value is reduced by a value (x) and, in this respect, assumes the value 3.3, wherein the unrestricted air supply is interrupted as soon as 3.4, the momentary sensor value 3.1, falls below the calculated value 3.3, or in that, if the evaluation electronics 2.3 establish that, as shown in Figure 4, the amount 4.3 by which the sensor signal alters in a freely determined time is larger than a freely selected value (y), a switching signal is likewise initiated.

2. Process according to Claim 1, characterised in that, in the case of sensors, which increase their electronic resistance when exposed to gas, the rules given in Claim 1 apply, _mutatis mutandis_, wherein, as shown in Figure 8, the integral 8.2 is above the sensor value 8.1 and the amount of alteration (y) is allocated to a positive gradient.

3. Process according to Claim 1, characterised in that the unrestricted air supply is reenabled by retraction of the switching command generated in Claims 1 and 2, if, as shown in Figure 3, the momentary sensor value 3.1 again assumes a greater value than the integral 3.3 which is reduced by the value (x).

4. Process according to Claim 1, characterised in that the unrestricted air supply is reenabled by retraction of the switching command generated in Claim 1 or 2, if, as shown in Figure 5, the tendency of the sensor signal 5.1 reverses at a point in time 5.4 owing to desorption, and the momentary sensor signal at this time is stored in the evaluation electronics, and increased by a fixed or proportional value to a switching level 5.5, wherein the abovementioned switching signal is initiated if the value of the momentary sensor signal exceeds the value 5.5. This applies to sensors which react to exposure to gas by reducing the sensor resistance.

5. Process according to Claim 4, characterised in that, in the case of a sensor which reacts to exposure to gas by increasing the sensor resistance, that which was stated in Claim 4 applies _mutatis mutandis_, wherein the level ratios are inverse.

6. Process according to Claim 1, characterised in that, as shown in Figure 7, the gradient is monitored after clearing the closing command at the point in time 7.7 and, in each case, the last valid quotient of the amount of alteration of the sensor level and a given time sector is stored.
   If the gradient 7.4 is reversed, the negative gradient D1 7.5 is summed, as an absolute value, with the absolute value of the positive gradient D2 7.7, wherein a clearing of the switching pulse occurs if the sum value of both gradients is larger than a given value D3. At the point in time 7.4, the sensor value which is reduced by a value (x) is set from the sensor values of a freely selected covered timespan 7.2, by means of the evaluation unit 2.3, to a new value, which results from the sensor value at the time point of the gradient reversal 7.4,

5

reduced by the value (x), wherein the new sensor values, detected after the point in time 7.4 are used to form a new integral 7.6. After the gradient reversal, the following applies as the switching condition:

D3 is greater than y

or

the momentary sensor value becomes less than the curve 7.6 which was newly defined above. If sensors are used which increase their resistance when exposed to gas, that which has been stated applies, _mutatis mutandis_, wherein the alterations in the level are, however, inverse.

7. Process according to Claim 1 - 6, characterised in that, as shown in Figure 8, the formation of a comparison level from the integrated value of the individual values of a freely definable covered timespan, reduced by a value x 8.2, is interrupted if the gradient of the momentary sensor value 8.4 becomes positive. If the gradient is positive, then the comparison value, 8.4 is formed of the momentary sensor value, minus a value x.
If sensors which increase their resistance when exposed to gas, are used, that which has been stated applies _mutatis mutandis_, wherein, however, the alterations in the level are inverse.

8. Process according to Claim 7, characterised in that the comparison level 8.2 results from the integral of sensor values over a determined timespan, minus a value x, and, after the reversal of the gradient, at the point in time 8.3, the duration of the integration is shortened. The normal, longer integration time is obtained again if the gradient 8.5 reaches a quotient which is smaller than a determined value.

9. Process according to one of the preceding Claims, characterised in that the features of one of the Claims are combined with another feature from one of the other Claims.

## Revendications

1. Procédé d'exploitation de signaux de détecteurs aux fins de produire des impulsions de commutation qui, par l'intermédiaire d'organes de réglage et d'arrivées d'air adaptés, agissent sur l'alimentation en air d'une chambre intérieure, de préférence de l'habitacle d'un véhicule automobile, de telle sorte que l'alimentation en air soit interrompue ou que l'air entrant passe dans un système de filtre avant d'arriver à la chambre intérieure, lorsqu'une concentration de gaz constituant un critère de commutation est atteinte, et que l'état d'origine soit rétabli, lorsque la qualité de l'air extérieur s'est améliorée, caractérisé par le fait que conformément à la figure 3, dans le cas de détecteurs dont la résistance électrique varie en présence de gaz, on forme, à partir de la valeur de détecteur 3.1 actuelle, une intégrale 3.2 sur un intervalle de temps (t), de laquelle on retranche une valeur (x) et qui prend ainsi la valeur 3.3, l'entrée libre de l'air étant interrompue dès que 3.4, la valeur de détecteur 3.1 actuelle, est inférieure à la valeur calculée 3.3 ou qu'un signal de commutation est également émis lorsque l'électronique d'exploitation 2.3 constate que, conformément à la figure 4, la valeur de la variation du signal 4.3 de détecteur à l'intérieur d'un intervalle de temps librement déterminé est supérieure à une valeur (y) librement déterminée.

2. Procédé selon la revendication 1, caractérisé par le fait que dans le cas de détecteurs dont la résistance électrique augmente en présence de gaz, on applique les règles mentionnées dans la revendication 1, l'intégrale 8.2 étant, conformément à la figure 8, supérieure à la valeur de détecteur 8.1 et la valeur de la variation (y) étant associée à un gradient positif.

3. Procédé selon la revendication 1, caractérisé par le fait que l'entrée libre d'air est rétablie par annulation de l'ordre de commutation produit dans les revendications 1 et 2 lorsque, conformément à la figure 3, le signal de détecteur 3.1 prend une valeur supérieure à l'intégrale 3.3 diminuée de la valeur (x).

4. Procédé selon la revendication 1, caractérisé par le fait que l'entrée libre d'air est rétablie par annulation de l'ordre de commutation produit dans la revendication 1 ou 2 lorsque, conformément à la figure 5, la tendance du signal de détecteurs 5.1, à l'instant 5.4, s'inverse par désorption et que le signal de détecteur actuel à cet instant est mémorisé dans l'électronique d'exploitation et est augmenté d'une valeur fixe, proportionnelle à un niveau de commutation 5.5, le signal de commutation mentionné étant déclenché lorsque la valeur du signal de détecteur actuel est supérieure à la valeur 5.5. Ceci vaut pour des détecteurs qui réagissent par une diminution de leur résistance du détecteur à la présence de gaz.

5. Procédé selon la revendication 4, caractérisé par le fait que ce qui a été indiqué à la revendication 4 vaut également pour un détecteur qui réagit par une augmentation de sa résistance à la présence de gaz, les rapports de niveau étant inversés.

6. Procédé selon la revendication 1, caractérisé par le fait que que conformément à la figure 7, après annulation de l'ordre de fermeture à l'instant 7.7, on surveille le gradient et on mémorise le dernier quotient de la valeur de la variation du niveau de signal et d'un intervalle de temps défini.

Lors d'une inversion du gradient 7.4, on ajoute le gradient négatif D1 7.5 sous forme de valeur absolue à la valeur absolue du gradient positif D2 7.7, une impulsion de commutation étant alors émise lorsque la somme des deux gradients est supérieure à une valeur D3 donnée. A l'instant 7.4 l'unité d'exploitation 2.3 amène la valeur de détecteur obtenue par intégration des valeurs de détecteur sur un intervalle de temps 7.2 antérieur librement défini, diminuée de la valeur (x), à une nouvelle valeur qui correspond à la valeur du détecteur à l'instant 7.4 de l'inversion du gradient, diminuée de la valeur (x), les nouvelles valeurs de détecteur déterminées à partir de l'instant 7.4 étant utilisées pour former une nouvelle intégrale 7.6. On a comme critère de commutation, après l'inversion du gradient,

soit D3 est supérieur à y

soit la valeur actuelle de détecteur est inférieure à la nouvelle courbe 7.6 telle que définie plus haut.

Lorsqu'on utilise des détecteurs dont la résistance augmente en présence de gaz, ce qui a été indiqué précédemment reste valable, les variations de niveau étant inversées.

7. Procédé selon l'une des revendications 1 à 6, caractérisé par le fait que conformément à la figure 8, l'établissement d'un niveau de référence à partir de la valeur d'intégration des valeurs individuelles à l'intérieur d'un intervalle de temps antérieur librement choisi diminuée de la valeur x 8.2 est retardé lorsque le gradient de la valeur de détecteur 8.4 actuelle devient positif. Si le gradient est positif, la valeur de référence 8.4 est composée de la valeur de détecteur actuelle diminuée d'une valeur x.

Lorsqu'on utilise des détecteurs dont la résistance augmente en présence de gaz, ce qui a été précédemment reste valable, les variations de niveau étant toutefois inversées.

8. Procédé selon la revendication 7, caractérisé par le fait que le niveau de référence 8.2 est obtenu à partir de l'intégrale des valeur de détecteur sur un intervalle de temps déterminé diminué d'une valeur x et que la durée d'intégration est réduite après l'inversion du gradient à l'instant 8.3. La durée d'intégration normale, plus longue, est rétablie lorsque le gradient 8.5 atteint un quotient qui est inférieur à une valeur déterminée.

9. Procédé selon au moins une des revendications précédentes, caractérisé par le fait que l'une des caractéristiques des revendications est combinée à une autre caractéristique des autres revendications.

EP 0 713 454 B1

Fig. 1

Fig. 2

Fig. 3

Fig. 4

Fig. 5

Fig. 6

## Fig. 7

## Fig. 8